(19) Europäisches Patentamt
European Patent Office
Office européen des brevets

(11) **EP 2 548 016 B1**

(12) **FASCICULE DE BREVET EUROPEEN**

(45) Date de publication et mention
de la délivrance du brevet:
**23.04.2014 Bulletin 2014/17**

(51) Int Cl.:
*G01N 33/36* (2006.01)     *G01N 3/08* (2006.01)
*G01N 3/32* (2006.01)     *G01N 3/40* (2006.01)

(21) Numéro de dépôt: **11712990.8**

(22) Date de dépôt: **11.03.2011**

(86) Numéro de dépôt international:
**PCT/FR2011/050493**

(87) Numéro de publication internationale:
**WO 2011/114044 (22.09.2011 Gazette 2011/38)**

(54) **PROCEDE ET DISPOSITIF DE CARACTERISATION DES PROPRIETES SENSORIELLES DE TISSUS**

VERFAHREN UND VORRICHTUNG ZUR CHARAKTERISIERUNG DER SENSORISCHEN EIGENSCHAFTEN VON STOFFEN

METHOD AND DEVICE FOR CHARACTERIZING SENSORY PROPERTIES OF FABRICS

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **19.03.2010 FR 1051982**

(43) Date de publication de la demande:
**23.01.2013 Bulletin 2013/04**

(73) Titulaires:
• **Ecole Centrale De Lyon
69134 Ecully Cedex (FR)**
• **Centre National de la Recherche Scientifique
75016 Paris (FR)**
• **Ecole Nationale d'Ingénieurs de Saint Etienne
42100 Saint Etienne (FR)**

(72) Inventeurs:
• **ZAHOUANI, Hassan
F-25000 Besançon (FR)**
• **VARGIOLU, Roberto
F-69390 Millery (FR)**

(74) Mandataire: **Thibault, Jean-Marc
Cabinet Beau de Loménie
51, Avenue Jean Jaurès
B.P. 7073
69301 Lyon Cédex 07 (FR)**

(56) Documents cités:
EP-B1- 0 899 555     WO-A1-2007/026143
FR-A3- 2 899 687     US-A1- 2006 036 410

# Description

La présente invention concerne le domaine de la caractérisation de propriétés sensorielles de matériaux et matières et notamment de tissus.

L'invention concerne plus particulièrement un procédé et un dispositif de caractérisation des propriétés sensorielles d'un tissu immitant la préhension du tissu avec la main.

Dans le domaine de l'invention, il est connu d'évaluer par simple toucher la qualité de tissus, en particulier pour l'industrie de la confection de luxe. En effet, La fabrication de tissus de luxe fait intervenir plusieurs étapes qui se superposent et une dérive peut avoir des conséquences économiques et d'images désastreuses pour l'industriel. Une non-conformité des tissus à pour effets la destruction de plusieurs kilomètres de tissus et/ou le retour client ou boutique de produits déjà confectionnés tels que des cravates ou des foulards par exemple.

De plus, l'évaluation des tissus par le toucher sert également aux fabricants de vêtements pour évaluer leurs différentes sources d'approvisionnement, leurs niveaux, le vieillissement des tissu, mais aussi comprendre a posteriori le choix consommateur qui déclenche l'achat d'un produit textile.

Pour réaliser l'évaluation des tissus qu'ils emploient, les industriels ont actuellement recours à des panelistes, c'est-à-dire des individus qui réalisent des tests de préhension et d'évaluation de la douceur des tissus à la main. Cependant cette réalité présente de nombreux inconvénients.

En effet, la sensation de toucher reste pour chaque personne subjective et tributaire de l'humeur et sensibilité du testeur et de sa qualité à reproduire des gestes identiques et à décrire la perception ressentie.

De plus, il est très difficile de remplacer les personnels panelistes par de nouveaux du fait du caractère subjectif de la sensation de toucher, qui ne peut concrètement ni s'apprendre ni se transmettre à l'identique d'une personne à une autre. Il faut donc que chaque paneliste se fasse sa propre formation sur le tas, en comparant des tissus conformes à des tissus non conformes aux critères de fabrication requis et en enregistrant pour chacun ses propres sensations de toucher particulières.

Face à ces inconvénients d'évaluation subjective de la qualité des tissus au toucher, les industriels recherchent depuis longtemps des outils de caractérisation objective et reproductible de la qualité et des propriétés sensorielles des tissus. Jusqu'à ce jour cependant aucune technique satisfaisante n'a semble-t-il été proposée.

Le but de la présente invention est de palier à ce manque et de fournir une technique de caractérisation des propriétés sensorielles des tissus d'une façon proche de la technique manuelle mais qui soit fiable et reproductible dans le temps.

A cette fin la présente invention propose en premier lieu un procédé de caractérisation des propriétés sensorielles d'un tissu, dans lequel on détermine simultanément la douceur et l'effort lié à la raideur d'un tissu par mesure du niveau vibratoire moyen $L_V$, défini par la formule $L_V = 20 \log V/Vo$ et de l'effort de traction tangentielle **Frt** engendrés lors d'une traction unidirectionnelle à vitesse constante d'un fragment de tissu au contact d'applicateurs sphériques reliés chacun à au moins un capteur de vibration et au moins un capteur d'effort, eux même reliés à un dispositif d'acquisition numérique.

La solution proposée est simple, elle mesure simultanément la douceur (mesure vibratoire) et la raideur (capteurs d'efforts) du tissu. Ces deux informations physiques sont similaires à celles ressenties par la main lors du toucher d'un tissu par préhension.

Le procédé de l'invention constitue une solution objective, fiable et reproductible de caractérisation des tissus par rapport aux tests humains. En effet, dans le cas de la fabrication de tissus de luxe, le procédé de l'invention permet de détecter toute dérive dans la fabrication par la mesure du rendu vibratoire Lv et des efforts procurés par les tests successifs ou en continu d'échantillons de tissus issus de la production là où les testeurs humains intègrent de petites dérives de toucher au fur et à mesure de la fabrication et où ce n'est que lorsque la dérive devient importante que la non-conformité du tissu est identifiée, mais alors avec des conséquences déjà lourdes en termes de fabrication défectueuse.

Le procédé proposé permet de suivre, d'évaluer et d'apporter des solutions pour corriger les dérives de fabrication. De plus, ce procédé permet d'établir une unique référence de contrôle qualité, qu'il est possible d'appliquer sur différents sites géographiques de production. Il est alors possible de suivre et de comparer les différentes productions de tissu à une échelle nationale ou internationale.

Selon une première caractéristique préférée du procédé de l'invention, le fragment de tissu est de forme régulière et on le tracte par son centre entre au moins trois applicateurs sphériques disposés en triangle et dépourvus de contact entre eux.

Selon une autre caractéristique préférée, la vitesse Vo de traction du fragment de tissu est constante et comprise entre 20 et 30 mm par seconde.

Avantageusement, les capteurs de vibration sont des accéléromètres permettant la mesure de la vibration du fragment de tissu au contact des applicateurs sphériques lors de la traction dudit fragment de tissu.

Selon un deuxième aspect, l'invention concerne également un dispositif de caractérisation des propriétés sensorielles d'un tissu. Ce dispositif permet une mise en oeuvre adéquate du procédé de l'invention. Il comporte un bâti annulaire définissant une ouverture sphérique centrale et sur lequel sont fixés des bras de mesure disposés à égale distance les uns des autres et s'étendant chacun suivant une direction verticale depuis le bâti annulaire sur lequel ils sont fixés, lesdits bras supportant chacun au moins un capteur d'effort et au moins un applicateur sphérique équipé d'un capteur de vibrations, les applicateurs sphériques délimitant entre eux une couverture coaxiale à celle définie par le bâti annulaire et au travers de laquelle peut être tracté un fragment de tissu au contact desdits applicateurs sphériques, les capteurs d'effort et les capteurs de vibration étant reliés à un dispositif d'acquisition numérique.

Selon une première caractéristique avantageuse, le dispositif de l'invention comporte trois bras portant chacun un applicateur sphérique, disposés en triangle à 120° les uns des autres.

De préférence, les applicateurs sphériques sont constitués de boules situées aux extrémités libres des bras de mesure solidaires du bâti annulaire.

De préférence encore, les boules constituant les applicateurs sont creuses, et avantageusement constituées de matière plastique ou de carbone.

Selon une autre caractéristique avantageuse du dispositif de l'invention, celui-ci comporte des moyens de guidage d'un fragment de tissu à caractériser au travers de l'ouverture délimitée entre les applicateurs sphériques.

Selon un mode de réalisation préféré, les moyens de guidage sont constitués de billes solidaires des applicateurs sphériques.

Dans ce mode de réalisation, chaque applicateur sphérique porte de préférence deux billes sensiblement opposées l'une de l'autre sur la surface de chaque applicateur, lesdites billes étant disposées de telle sorte que chaque bille d'un applicateur soit située sensiblement sur une même droite verticale avec une bille de chacun des applicateurs voisins.

Toujours selon l'invention, le dispositif comporte également un arbre de traction automatique d'un fragment de tissu au travers de l'ouverture ménagée entre les applicateurs sphériques. Avanatageusement, cet arbre de traction automatique peut être actionné par un mécanisme programmable.

Diverses autres caractéristiques ressortent de la description faite ci-dessous en référence aux dessins annexés qui montrent, à titre d'exemples non limitatifs, des formes de réalisation de l'objet de l'invention.

- la **figure 1** représente en perspective un dispositif de caractérisation des propriétés sensorielles d'un tissu conforme à la présente invention dans un mode préféré de réalisation,
- la **figure 2** représente le dispositif de la figure 1 en vue de dessus,
- la **figure 3** représente une machine de test de caractérisation des propriétés sensorielles d'un tissu comprenant un dispositif tel que représenté sur les figures 1 et 2,
- la **figure 4** représente les diagrammes de signature de différents tissus obtenus selon le procédé de caractérisation de l'invention à l'aide du dispositif des figures 1 et 2,
- la **figure 5** représente trois diagrammes obtenus pour pour trois lots d'un tissu soumis au procédé de l'invention afin de déterminer la conformité de ces lots de tissus,
- la **figure 6** représente les résultats d'un essai de caractérisation de la douceur de mouchoirs par comparaison à une référence établie par mise en oeuvre du procédé de caractérisation de l'invention.

La présente invention propose un procédé et un dispositif dédiés à la caractérisation des propriétés sensorielles de tissus, et plus généralement de matières souples tissées ou non tissées. Par propriétés sensorielles on entend particulièrement au sens de la présente invention les propriétés de douceur ou raideur de telles matières. Ces propriétés sont particulièrement importantes à évaluer dans l'industrie de la confection, notamment de luxe, afin de garantir une qualité de fabrication des produits constante pour la clientèle.

Le procédé et le dispositif de l'invention procurent une solution technique fiable et reproductible pour l'évaluation et la caractérisation des propriétés sensorielles de tissus qui, pour la première fois, permettent d'envisager le remplacement de panélistes humains pour réaliser le contrôle qualité des tissus employés.

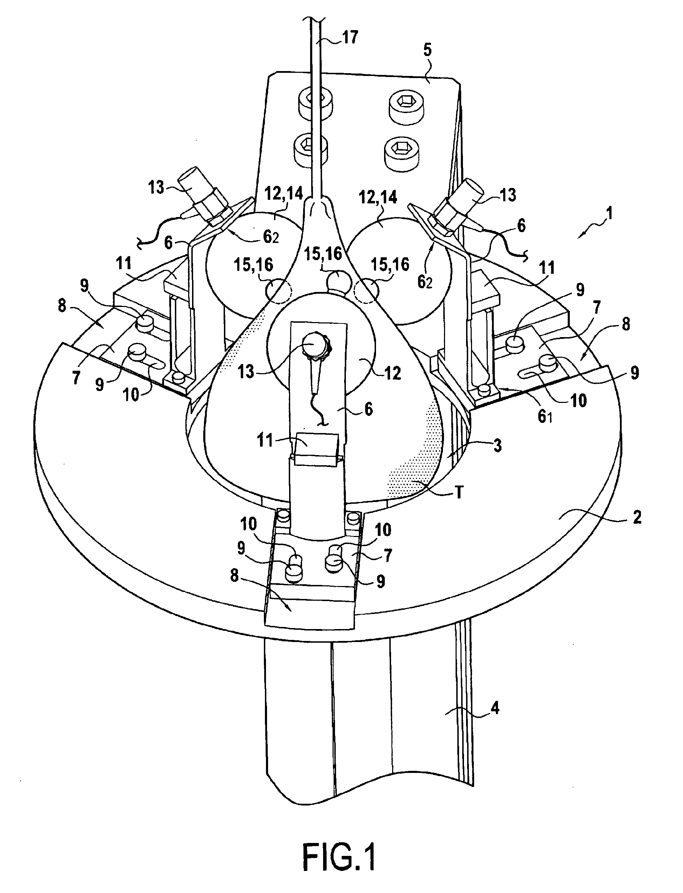

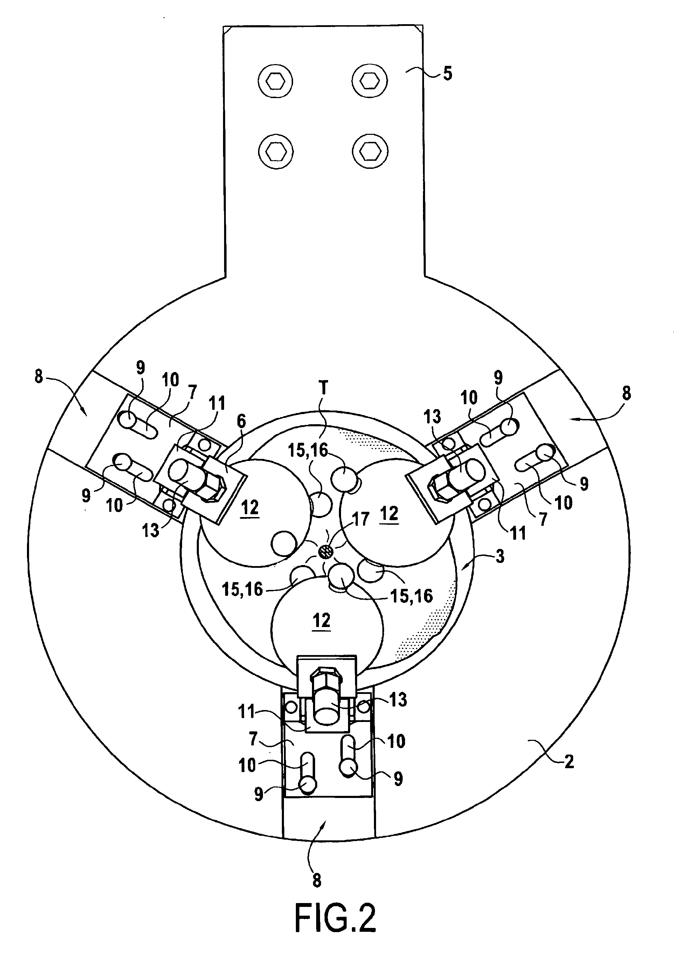

Un exemple de réalisation du dispositif de l'invention est représenté aux **figures 1** et **2**. Ce dispositif **1** comporte un bâti annulaire **2** horizontal définissant une ouverture sphérique centrale **3**. Le bâti **2** est monté solidairement sur un pied **4** de stabilisation vertical par l'intermédiaire d'une platine rectangulaire **5** s'étendant depuis la périphérie du bâti annulaire **2**, ladite platine **5** étant dans l'exemple représenté sur les **figures 1** et **2** boulonnée sur le pied **4**.

Le bâti annulaire **2** supporte trois bras de mesure **6** disposés à égale distance suivant une répartition angulaire à 120 ° les uns des autres autour de l'ouverture centrale **3**. Ces bras de mesure **6** s'étendent chacun suivant une direction verticale perpendiculaire à la surface du bâti annulaire **2**. Ils sont avantageusement fixés sur chacun sur une platine **7** réglable par coulissement dans des glissières **8** formées radialement dans la masse du bâti **2**. Sur les **figures 1** et **2**, les platines **7** sont parallélépipédique et coulissent dans des glissières **8** de section tranversale rectangulaires. Le réglage de la position desdites platines **7** et donc des bras de mesure **6** s'effectue par des vis de réglages **9** logées dans des coulisses **10** pratiquées dans les platines **7**. Toutefois, on peut bien entendu envisager d'autres formes de réglage en position des bras de mesure **6** sur le bâti **2**. En particulier, les platines **7** peuvent être en queue d'aronde dans des glissières **8** de section trapézoidale ou encore prendre la forme d'un système de coulissement en té inversé des platines dans les glissières, le blocage en position étant réalisé dans tous les cas par des vis de réglage comme dans l'exemple présenté.

Les bras de mesure **6** sont avantageusement constitués d'une lame métallique flexible par rapport à sa base d'ancrage **6₁** sur la platine **7** et supportent chacun au moins un capteur d'effort **11** sur leur paroi dorsale de préférence et au moins un applicateur sphérique **12** au contact d'un capteur de vibration **13** à leur extrémité supérieure libre **6₂**. Les applicateurs sphériques délimitent entre eux une couverture coaxiale à l'ouverture **3** définie par le bâti annulaire **2** et au travers de laquelle peut être tracté un fragment de tissu **T** au contact desdits applicateurs sphériques **12**.

Enfin, les capteurs d'effort **11** et les capteurs de vibration **13** sont reliés à un dispositif d'acquisition numérique non représenté sur les figures qui peut être composé avantageusement d'un ordinateur équipé d'un programme d'acquisition de mesures d'efforts et de vibrations et d'analyse numérique de ces données.

Selon l'invention, les capteurs de vibrations **13** sont des accéléromètres, fixés sur l'extrémité libre **6₂** des bras de mesure, et dont le capteur est en contact des applicateurs sphériques **12**, également fixés chacun sur l'extrémité libre **6₂** des bras **6**.

Les capteurs d'effort **11** quant à eux ne sont fixés que sur la lame flexible formant chaque bras afin d'enregistrer la déformation de ces bras lors du passage d'un tissu **T** entre les applicateurs sphériques **12** et de mesurer ainsi l'effort tangentiel appliqué aux applicateurs sphériques **12** et bras de mesure **6** comme il sera décrit par la suite.

Comme cela ressort des **figures 1** et **2**, les applicateurs sphériques **12** sont avantageusement constitués, de façon préférée, par des boules **14** creuses, telles des balles de tennis de table, ou de golf, fixées solidairement aux extrémités libres des bras de mesure **6**, ces boules **14** étant réalisées soit en une matière plastique telle que du polyuréthane soit en carbone pour une grande rigidité et une bonne transmission des vibrations, et donc sensibilité, et une grande résistance.

Additionnellement, le dispositif **1** de l'invention comporte également des moyens de guidage **15** d'un fragment de tissu **T** à caractériser au travers de l'ouverture délimitée entre les applicateurs sphériques **12**. Avantageusement, ces moyens de guidage consistent en des billes **16** d'acier collées sur les applicateurs sphériques **12** au nombre de deux par applicateur disposées de telle sorte que chaque bille **16** d'un applicateur soit située sensiblement sur une même droite verticale qu'une bille de chacun des applicateurs voisins.

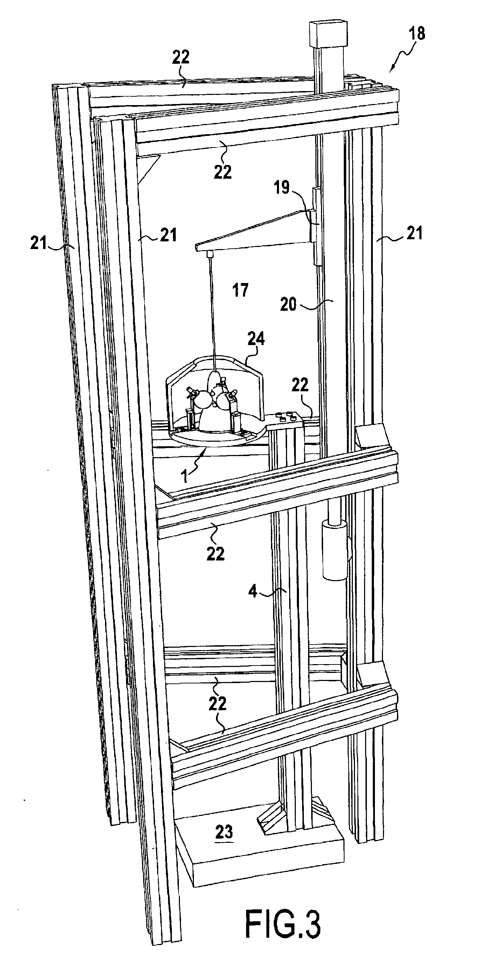

Le dispositif **1** de l'invention comporte enfin un arbre **17** de traction automatique d'un fragment de tissu **T** au travers de l'ouverture **3** au centre du bâti **2** et de celle ménagée entre les applicateurs sphériques **12**. Cet arbre de traction **17** est équipé d'un capteur d'effort normal relié au dispositif d'acquisition numérique pour mesurer l'effort de traction des tissus **T** au contact des applicateurs **12** et est de façon préférée, comme représenté sur la **Figure 3**, solidaire d'un portic **18** de traction linéaire à vitesse constante d'un fragment de tissu T à caractériser, ledit portic **18** comprenant un mécanisme programmable comprenant un chariot **19** coulissant dans un rail vertical **20** sous l'action d'un dispositif moteur, par exemple à courroie, logé dans le rail et piloté par un logiciel.

Dans l'exemple de la **figure 3**, le portic **18** forme un trepied stable de traction comprenant trois montants **21** verticaux placés en triangle, par exemple constitués de poutrelles d'aluminium, et reliés deux à deux par un jeu de six traverses **22**, le montant formant le sommet du triangle supportant le rail vertical **20** de traction, luimême recevant le chariot **19** de traction solidaire de l'arbre de traction **17**.

Dans cette configuration de portic **18**, le dispositif **1** de caractérisation de l'invention peut être placé sur son pied **4**, stabilisé de préférence par un ancrage massique **23** tel qu'un bloc de béton, au milieu du portic **18** entre les trois montants de celui-ci de telle sorte que le centre de l'orifice central **3** du bâti annulaire **2** soit localisé à l'aplomb du bras de traction **17** pour ensuite effectuer des tests de caractérisation de fragments de tissus **T** selon la méthode décrite ci-après. Il convient également de noter qu'avantageusement comme représenté sur la **figure 3**, le dispositif **1** peut être couvert sous une cloche **24** pour le protéger lors des tests de perturbations extérieures.

Le dispositif **1** de l'invention tel que décrit précédemment et représenté aux **figures 1** à **3** a été conçu et développé par les inventeurs pour caractériser de façon simple, rapide, fiable et reproductible les propriétés sensorielles de douceur et/ou de raideur de matières souples tels que des tissus pour déterminer notamment leur conformité à un ou des standards de qualité en vigueur dans l'industrie de la confection.

Pour ce faire, selon l'invention, on découpe dans chaque échantillon de tissu à caractériser un fragment de tissu **T** de forme carré, par exemple de 20x20 cm, qui est ensuite fixé en son centre à l'arbre de traction **17** du dispositif **1**. pour ce faire, l'extrémité de l'arbre de traction **17** porte un aimant, contre lequel le tissu T est plaqué par un pion métallique ou un deuxième aimant passé de l'autre côté du tissu T en face de l'extrémité de l'arbre **17**. Ceci évite le perçage du tissu. L'arbre de traction **17** est ensuite tiré par le chariot de traction **19** suivant une direction unique verticale à vitesse constante entre les trois applicateurs sphériques **12** portés par les bras de mesure **6** sur le bâti.

Lors du passage du tissu **T** entre les applicateurs **12**, le frottement du tissu sur les applicateurs **12** génère un signal de vibration **L_v** capté par les accéléromètres et enregistré par le dispositif d'acquisition. Cette information traduit la douceur du tissu. De plus, le passage du tissu provoque l'écartement des applicateurs **12** et des bras de mesure **6**. Cette déformation est mesurée par les trois capteurs d'effort qui génèrent un signal **Frt** de déformation enregistré par le dispositif d'acquisition numérique. Cette information traduit l'effort de raideur du tissu **T** tel qu'il serait perçu par la préhension de la main d'un panéliste humain ou d'un consommateur lambda.

A partir de ces mesures, on calcule respectivement ensuite le niveau vibratoire moyen **L_v** et l'effort de traction tangentielle **Frt** engendrés lors de la traction du fragment de tissu **T** au contact des applicateurs sphériques **12**. Le niveau vibratoire moyen **L$_v$**, qui s'exprime en décibels (dB), est définit par la formule

$$\mathbf{L_v} = 20\log\frac{V}{V_0},$$ où V$_0$ est la vitesse de traction constante du chariot et de l'arbre de traction **17** sur le fragment de tissu **T**. Cette vitesse V$_0$ est comprise de préférence entre 20 et 30 mm par seconde. La vitesse V quant à elle est la vitesse mesurée par les accéléromètres au contact des boules formant les applicateurs sphériques lors du passage du fragment de tissu **T**.

A partir de ces deux valeurs de **L$_v$** et de **Frt** il est ensuite possible de qualifier la qualité du tissu en en fonction de sa douceur (**L$_v$**) et de sa raideur (**Frt**) par comparaison avec une signature du tissu testé, cette signature étant représentée par un diagramme x=f(y) pouvant repésenté **L$_v$=f(Frt) ou Frt=f(L$_v$)** établi par régression linéaire préalablement obtenu par calibration, à l'aide d'un nombre de mesures fini sur différents tissus de qualité connue, du dispositif de caractérisation et notamment du programme d'acquisition et d'analyse numérique exploitant les valeurs mesurées par les capteurs de vibration et d'effort.

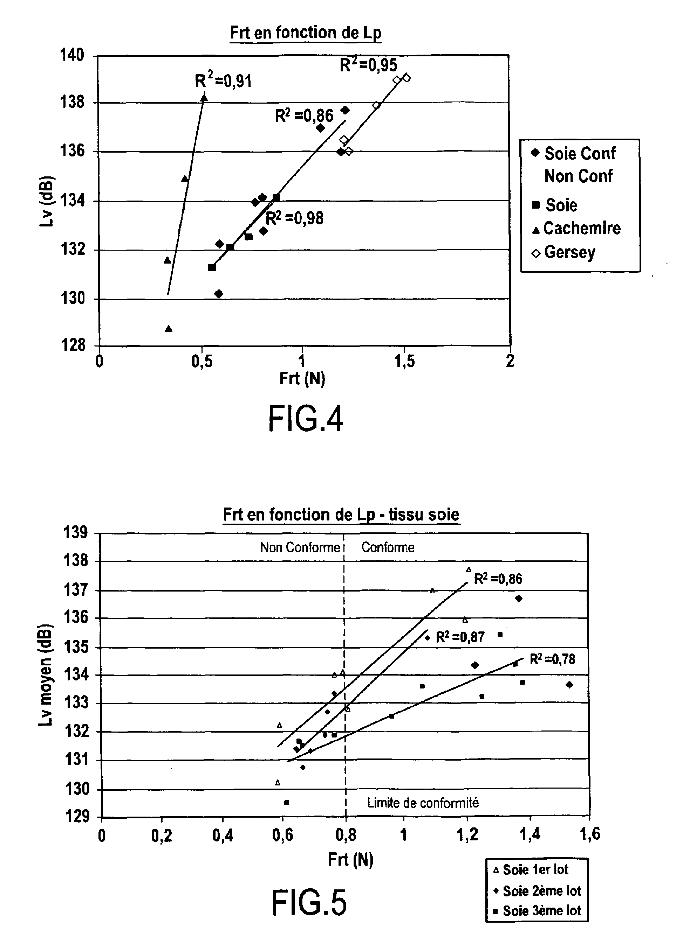

La **figure 4** représente différentes signatures de tissus tels que la soie, le cachemire et le gersey, obtenues par la mise en oeuvre du procédé de l'invention sur le dispositif représenté aux **figures 1** à **3**. Sur la **figure 4**, il convient de noter que pour un tissu donné, plus la valeur de **L$_v$** est faible plus le tissu est doux, plus la valeur de **Frt** est grande plus le tissu est raide. On peut ainsi constater, de façon assez logique au regard d'une perception commune manuelle des tissus en général, que l'effort **Frt** transcrivant la raideur d'un tissu augmente avec le niveau vibratoire moyen mesuré **L$_v$**, et donc que plus un tissu est raide, moins il est doux.

Une fois les signatures de tissus établies, il est possible de déterminer simplement et rapidement la qualité d'un tissu d'un lot ou provenance quelconque pour déterminer par exemple non seulement ses propriétés de douceur et de raideur mais surtout évaluer, notamment dans l'industrie de la confection, si ce tissu est conforme aux critères de qualité requis pour la fabrication. Il suffit pour cela de déterminer les valeurs de douceur (**L$_v$**) et raideur (**Frt**) limites en deça et/ou au dela desquelles un tissu ne peut être considéré comme conforme pour une fabrication, puis ensuite de simplement mesurer à l'aide du dispositif de l'invention les valeurs de **L$_v$** et **Frt** pour chaque tissu à caractériser et reporter les valeurs de mesures sur le diagramme de signature du tissu testé pour déterminer si en fonction des valeurs mesurées les échantillons de tissus testés sont conformes ou non.

La **figure 5** représente ainsi un exemple d'essai de conformité de trois lots de soie de qualité inconnue. La limite de conformité ayant été fixée dans l'exemple présenté à une valeur de raideur de 0,8 N, on peut ainsi aisément distinguer directement sur le diagramme les échantillons conformes des échantillons non conformes.

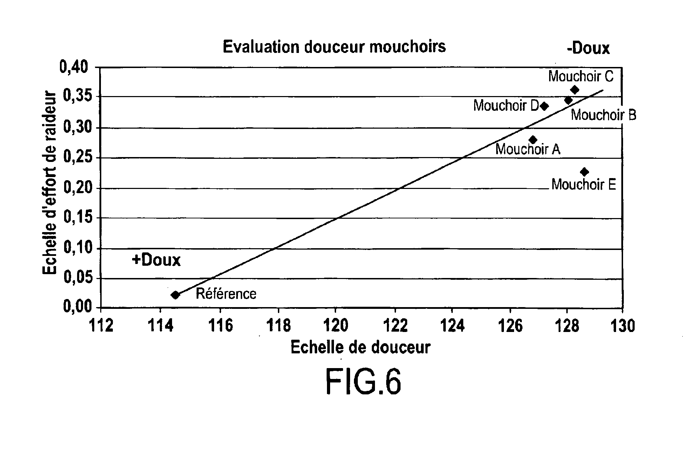

Le procédé et le dispositif de l'invention peuvent également servir à évaluer la douceur/raideur de matières non tissées telle que de la ouate de cellulose pour la réalisation de mouchoirs en papier. La **figure 6** représente ainsi les valeurs recueillies lors d'un test d'évaluation de douceur de cinq lots différents de mouchoirs. Sur cette figure contrairement aux **figures 4** et **5**, le diagramme est de type **Frt=f(L$_v$)**. On peut ainsi constater que les cinq lots de mouchoirs testés sont beaucoup moins doux que la référence.

La classification des tissus tissés ou non tissés suivant un diagramme x=f(y) des paramètres L$_v$ et Frt permet d'établir une hiérarchisation des tissus suivant une information de douceur et d'effort de raideur des tissus tel que cette information pourrait être perçue par la main.

Dans l'exemple de la **figure 6**, les lots de tissus testés sont de trois épaisseurs différentes. Les résultats montrent que respectivement les lots du plus doux au moins doux sont A, D, B et C. Les mouchoirs du lot E quant à eux sont trop éloignés de la référence pour être considérés conformes. Ce lot E est un lot de mouchoirs "discount", comportant deux épaisseurs de tissus, alors que les lots A,B,C,D sont tous constitués d'au moins 3 épaisseurs de tissus.

En conclusion pour une même épaisseur de mouchoir le procédé de fabrication du lot A permet d'obtenir la sensation de toucher par préhension avec la main la plus douce par rapport aux autres lots.

L'invention n'est pas limitée aux exemples décrits et représentés car diverses modifications peuvent y être apportées sans sortir de son cadre.

## Revendications

1. Procédé de caractérisation des propriétés sensorielles d'un tissu (**T**), dans lequel on détermine simultanément la douceur et l'effort lié à la raideur d'un tissu par mesure du niveau vibratoire moyen **L$_v$**, définit

   par la formule $\mathbf{L_v} = 20\log\frac{V'}{V_o}$ V étant la vitesse mesurée par les accéléromètres au contact des boules formant les applicateurs sphériques lors du passage du fragment de tissu T et V$_0$ étant la vitesse de traction constante du chariot et de l'arbre de traction (17) sur le fragment de tissu T, et de l'effort de traction tangentielle **Frt** engendrés lors d'une traction unidirectionnelle à vitesse constante d'un fragment de tissu (**T**) au contact d'applicateurs sphériques (**12**) solidaires chacun d'au moins un capteur de vibrations (**13**) et au moins un capteur d'effort (**11**), eux même reliés à un dispositif d'acquisition numérique.

**2.** Procédé selon la revendication 1, dans lequel le fragment de tissu (**T**) est de forme régulière et on le tracte par son centre entre au moins trois applicateurs sphériques (**12**) disposés en triangle et dépourvus de contact entre eux.

**3.** Procédé selon l'une des revendications 1 ou 2, dans lequel la vitesse $V_o$ de traction est constante et comprise entre 20 et 30 mm par seconde.

**4.** Procédé selon l'une des revendications 1 à 3, dans lequel les capteurs de vibrations (**13**) sont des accéléromètres permettant la mesure de la vibration du fragment de tissu (**T**) au contact des applicateurs sphériques (**12**) lors de la traction dudit fragment de tissu.

**5.** Dispositif (**1**) de caractérisation des propriétés sensorielles d'un tissu (**T**), **caractérisé en ce qu'**il comporte un bâti annulaire (**2**) définissant une ouverture sphérique centrale (**3**) et sur lequel sont fixés des bras de mesure (**6**) disposés à égale distance les uns des autres et s'étendant chacun suivant une direction verticale depuis le bâti annulaire (**2**) sur lequel ils sont fixés, lesdits bras de mesure (**6**) supportant chacun au moins un capteur d'effort (**11**) et au moins un applicateur sphérique (**12**) équipé d'un capteur de vibrations (**13**), les applicateurs sphériques (**12**) délimitant entre eux une couverture coaxiale à celle définie par le bâti annulaire (**2**) et au travers de laquelle peut être tracté un fragment de tissu (**T**) au contact desdits applicateurs sphériques, les capteurs d'effort et les capteurs de vibration étant reliés à un dispositif d'acquisition numérique.

**6.** Dispositif de caractérisation selon la revendication 5, **caractérisé en ce qu'**il comporte trois bras de mesure (**6**) portant chacun un applicateur sphérique (**12**) et disposés angulairement sur le bâti (**2**) à 120° les uns des autres.

**7.** Dispositif de caractérisation selon la revendication 5 ou 6, **caractérisé en ce que** les applicateurs sphériques (**12**) sont constitués de boules (**14**) situées aux extrémités libres (**6₂**) des bras de mesure (**6**) solidaires du bâti annulaire (**2**).

**8.** Dispositif de caractérisation selon la revendication 7, **caractérisé en ce que** les boules (**14**) constituant les applicateurs sphériques (**12**) sont creuses.

**9.** Dispositif selon l'une des revendications 5 à 8, **caractérisé en ce que** les applicateurs sphériques (**12**) sont formés par des boules (**14**) de matière plastique ou de carbone.

**10.** Dispositif de caractérisation selon l'une des revendications 5 à 9, **caractérisé en ce qu'**il comporte des moyens de guidage (**15**) d'un fragment de tissu (**T**) à caractériser au travers de l'ouverture délimitée entre les applicateurs sphériques (**12**).

**11.** Dispositif de caractérisation selon l'une des revendications 5 à 10, **caractérisé en ce que** les moyens de guidage (**15**) sont constitués de billes (**16**) solidaires des applicateurs sphériques (**12**).

**12.** Dispositif de caractérisation selon l'une des revendications 5 à 11, **caractérisé en ce que** chaque applicateur sphérique (**12**) porte deux billes (**16**) sensiblement opposée l'une de l'autre sur la surface de chaque applicateur, lesdites billes étant disposées de telle sorte que chaque bille d'un applicateur soit située sensiblement sur une même droite verticale avec une bille de chacun des applicateurs sphériques voisins.

**13.** Dispositif de caractérisation selon l'une des revendications 5 à 12, **caractérisé en ce qu'**il comporte un arbre de traction (**17**) automatique d'un fragment de tissu (**T**) au travers de l'ouverture ménagée entre les applicateurs sphériques (**12**).

**14.** Dispositif de caractérisation selon la revendication 13, **caractérisé en ce que** l'arbre de traction (**17**) automatique est actionné par un mécanisme programmable.

**15.** Dispositif de caractérisation selon la revendication 14, **caractérisé en ce que** le mécanisme programmable comporte un chariot (**19**) coulissant dans un rail vertical (**20**) supporté fixement par un portic (**18**), ledit chariot tirant l'arbre de traction (**17**) du fragment de tissu (**T**).

**Patentansprüche**

**1.** Verfahren zur Charakterisierung der sensorischen Eigenschaften eines Gewebes (T), wobei gleichzeitig die Geschmeidigkeit und die mit der Steifigkeit verbundene Kraft eines Gewebes durch Messung des mittleren Schwingungspegels $L_V$, definiert durch

die Formel $L_V = 20 \log \dfrac{V}{V_O}$, wobei V die Geschwindigkeit ist, die durch die Beschleunigungsmesser, welche mit den die Kugelapplikatoren bildenden Kugeln in Kontakt sind, während des Durchgangs des Gewebestücks T gemessen wird, und wobei $V_0$ die konstante Ziehgeschwindigkeit des Schlittens und der Ziehwelle (17) an dem Gewebestück T ist, sowie der tangentialen Zugkraft Frt bestimmt werden, die erzeugt werden während eines in einer Richtung wirkenden Ziehens mit konstanter Geschwindigkeit eines Gewebestücks (T), das mit Kugelapplikatoren (12) in Kontakt ist, die jeweils mit wenigstens einem Schwingungssensor (13) und wenigstens einem Kraftsensor (11) fest verbunden sind, welche selbst mit einer digitalen Erfassungseinrichtung verbunden sind.

2. Verfahren nach Anspruch 1, wobei das Gewebestück (T) eine gleichmäßige Form aufweist und es mit seiner Mitte zwischen wenigstens drei Kugelapplikatoren (12), die im Dreieck angeordnet sind und keinen Kontakt untereinander aufweisen, gezogen wird.

3. Verfahren nach einem der Ansprüche 1 oder 2, wobei die Ziehgeschwindigkeit $V_o$ konstant ist und zwischen 20 und 30 mm pro Sekunde beträgt.

4. Verfahren nach einem der Ansprüche 1 bis 3, wobei die Schwingungssensoren (13) Beschleunigungsmesser sind, die die Messung der Schwingung des Gewebestücks (T) in Kontakt mit den Kugelapplikatoren (12) beim Ziehen des Gewebestücks ermöglichen.

5. Vorrichtung (1) zur Charakterisierung der sensorischen Eigenschaften eines Gewebes (T), **dadurch gekennzeichnet, dass** sie ein ringförmiges Gestell (2) umfasst, das eine mittlere kugelförmige Öffnung (3) definiert und an dem Messarme (6) befestigt sind, die in gleichem Abstand voneinander angeordnet sind und sich jeweils von dem ringförmigen Gestell (2) aus, an dem sie befestigt sind, in einer vertikalen Richtung erstrecken, wobei die Messarme (6) jeweils wenigstens einen Kraftsensor (11) sowie wenigstens einen Kugelapplikator (12), welcher mit einem Schwingungssensor (11) ausgestattet ist, tragen, wobei die Kugelapplikatoren (12) zwischen sich eine Öffnung begrenzen, die zu der durch das ringförmige Gestell (2) definierten koaxial ist und durch die hindurch ein Gewebestück (T) in Kontakt mit den Kugelapplikatoren gezogen werden kann, wobei die Kraftsensoren und die Schwingungssensoren mit einer digitalen Erfassungseinrichtung verbunden sind.

6. Charakterisierungsvorrichtung nach Anspruch 5, **dadurch gekennzeichnet, dass** sie drei Messarme (6) umfasst, die jeweils einen Kugelapplikator (12) tragen und an dem Gestell (2) winkelmäßig 120° voneinander angeordnet sind.

7. Charakterisierungsvorrichtung nach Anspruch 5 oder 6, **dadurch gekennzeichnet, dass** die Kugelapplikatoren (12) von Kugeln (14) gebildet sind, die sich an den freien Enden ($6_2$) der Messarme (6), welche mit dem ringförmigen Gestell (2) fest verbunden sind, befinden.

8. Charakterisierungsvorrichtung nach Anspruch 7, **dadurch gekennzeichnet, dass** die die Kugelapplikatoren (12) bildenden Kugeln (14) hohl sind.

9. Vorrichtung nach einem der Ansprüche 5 bis 8, **dadurch gekennzeichnet, dass** die Kugelapplikatoren (12) durch Kugeln (14) aus Kunststoff oder aus Kohlenstoff gebildet sind.

10. Charakterisierungsvorrichtung nach einem der Ansprüche 5 bis 9, **dadurch gekennzeichnet, dass** sie Mittel zum Führen (15) eines zu charakterisierenden Gewebestücks (T) durch die zwischen den Kugelapplikatoren (12) begrenzte Öffnung hindurch umfasst.

11. Charakterisierungsvorrichtung nach einem der Ansprüche 5 bis 10, **dadurch gekennzeichnet, dass** die Führungsmittel (15) von kleinen Kugeln (16), welche mit den Kugelapplikatoren (12) fest verbunden sind, gebildet sind.

12. Charakterisierungsvorrichtung nach einem der Ansprüche 5 bis 11, **dadurch gekennzeichnet, dass** jeder Kugelapplikator (12) zwei im Wesentlichen voneinander abgewandte kleine Kugeln (16) auf der Oberfläche eines jeden Applikators umfasst, wobei die kleinen Kugeln derart angeordnet sind, dass jede kleine Kugel eines Applikators mit einer kleinen Kugel eines jeden der benachbarten Kugelapplikatoren im Wesentlichen auf einer gleichen vertikalen Geraden gelegen ist.

13. Charakterisierungsvorrichtung nach einem der Ansprüche 5 bis 12, **dadurch gekennzeichnet, dass** sie eine Welle zum automatischen Ziehen (17) eines Gewebestücks (T) durch die zwischen den Kugelapplikatoren (12) ausgebildete Öffnung hindurch umfasst.

14. Charakterisierungsvorrichtung nach Anspruch 13, **dadurch gekennzeichnet, dass** die automatische Ziehwelle (17) durch einen programmierbaren Mechanismus betätigt wird.

15. Charakterisierungsvorrichtung nach Anspruch 14, **dadurch gekennzeichnet, dass** der programmierbare Mechanismus einen Schlitten (19), der in einer durch einen Rahmen (18) fest getragenen vertikalen Schiene (20) gleitet, umfasst, wobei der Schlitten die Welle zum Ziehen (17) des Gewebestücks (T) zieht.

**Claims**

1. A method for characterizing sensorial properties of a fabric (**T**), wherein the softness and the force related to the stiffness of a fabric are determined simultaneously by measuring the average vibration level $L_v$, defined by the formula $L_v = 20\log\dfrac{V}{V_0}$, V being the velocity measured by the accelerometers upon contact with the balls forming the spherical applicators during the passage of the fabric fragment (**T**) and $V_0$ being the constant traction velocity of the carriage and of the traction shaft (**17**) on the fabric fragment (**T**) and of the tangential traction force **Frt** generated during a unidirectional connection at constant velocity of a fabric fragment (**T**) upon contact with spherical applicators (**12**) each are secured to at least a one vibration sensor (**13**) and at least one force sensor (**11**), those cells connected to a digital acquisition device.

2. The method according to claim 1, wherein the fabric fragment (**T**) is of a regular shape and is towed through its center between at least three spherical applicators (**12**) positioned in a triangle and without any contact with each other.

3. The method according to one of claims 1 or 2, wherein the traction velocity $V_0$ is constant and comprised between 20 and 30 mm per second.

4. The method according to one of claims 1 to 3, wherein the vibration sensors (**13**) are accelerometers allowing measurement of the vibration of the fabric fragment (**T**) upon contact with the spherical applicators (**12**) and during traction of said fabric fragment.

5. A device (**1**) for characterizing sensorial properties of a fabric (**T**), **characterized in that** it includes an annular frame (**2**) defining a central spherical aperture (**3**) and on which are attached measurement arms (**6**) positioned at an equal distance from each other and each extending along a vertical direction from the annular frame (**2**) on which they are attached, said measurement arms (**6**) each supporting at least one force sensor (**11**) and at least one spherical applicator (**12**) equipped with a vibration sensor (**13**), the spherical applicators (**12**) delimiting between them a cover coaxial with the one defined by the annular frame (**2**) and through which a fabric fragment (**T**) upon contact with said spherical applicators may be towed, the force sensors and the vibration sensors being connected to a digital acquisition device.

6. The characterization device according to claim 5, **characterized in that** it includes three measurement arms (**6**) each bearing a spherical applicator (**12**) and angularly positioned on the frame (**2**) at 120° from each other.

7. The characterization device according to claim 5 or 6, **characterized in that** the spherical applicators (**12**) consist of balls (**14**) located at the free ends (**6₂**) of the measurement arms (**6**) secured to the annular frame (**2**).

8. The characterization device according to claim 7, **characterized in that** the balls (**14**) forming the spherical applicators (**12**) are hollow.

9. The device according to one of claims 5 to 8, **characterized in that** the spherical applicators (**12**) are formed by balls (**14**) of plastic material or carbon.

10. The characterization device according to one of claims 5 to 9, **characterized in that** it includes means (**15**) for guiding a fabric fragment (**T**) to be characterized through the aperture delimited between the spherical applicators (**12**).

11. The characterization device according to one of claims 5 to 10, **characterized in that** the guiding means (**15**) consist of beads (**16**) secured to the spherical applicators (**12**).

12. The characterization device according to one of claims 5 to 11, **characterized in that** each spherical applicator (**12**) bears two beads (**16**) substantially opposite each other on the surface of each applicator, said beads being positioned so that each bead of an applicator is substantially located on a same vertical line with a bead of each of the neighboring spherical applicators.

13. The characterization device according to one of claims 5 to 12, **characterized in that** it includes a shaft (**17**) for automatic traction of a fabric fragment (**T**) or through the aperture made between the spherical applicators (**12**).

14. The characterization device according to claim 13, **characterized in that** the automatic traction shaft (**17**) is actuated by a programmable mechanism.

15. The characterization device according to claim 14, **characterized in that** the programmable mechanism includes a carriage (**19**) sliding on a vertical rail (**20**) fixedly supported by a gantry (**18**), said carriage pulling the shaft (**17**) for traction of the fabric fragment (**T**).

**FIG.1**

FIG.2

FIG.3

## Frt en fonction de Lp

FIG.4

## Frt en fonction de Lp - tissu soie

FIG.5

**Evaluation douceur mouchoirs**                    **-Doux**

FIG.6